(19) Europäisches Patentamt
European Patent Office
Office européen des brevets

(11) **EP 3 196 342 B1**

(12) **EUROPEAN PATENT SPECIFICATION**

(45) Date of publication and mention
of the grant of the patent:
**09.09.2020 Bulletin 2020/37**

(51) Int Cl.:
*D01D 5/253* (2006.01)      *D01D 5/24* (2006.01)
*D04H 1/56* (2006.01)       *D04H 1/4291* (2012.01)
*D04H 1/559* (2012.01)      *A61F 13/47* (2006.01)
*D01F 6/06* (2006.01)       *A61F 13/49* (2006.01)
*A61F 13/53* (2006.01)

(21) Application number: **15830750.4**

(22) Date of filing: **06.08.2015**

(86) International application number:
**PCT/KR2015/008263**

(87) International publication number:
**WO 2016/021968 (11.02.2016 Gazette 2016/06)**

(54) **MODIFIED CROSS-SECTION HOLLOW FIBER, AND FIBER ASSEMBLY USING SAME**

HOHLFASER MIT MODIFIZIERTEM QUERSCHNITT UND FASERANORDNUNG DAMIT

FIBRE CREUSE À SECTION TRANSVERSALE MODIFIÉE, ET ENSEMBLE DE FIBRES L'UTILISANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.08.2014   KR 20140100788
02.09.2014   KR 20140116331
29.10.2014   KR 20140148469
03.04.2015   KR 20150047576
17.07.2015   KR 20150101874**

(43) Date of publication of application:
**26.07.2017   Bulletin 2017/30**

(73) Proprietor: HUVIS Co., Ltd.
**Seoul 135-820 (KR)**

(72) Inventors:
• **KWON, Oh-hyuk
Daejeon 34083 (KR)**
• **LEE, Yun-jeong
Busan 47109 (KR)**
• **KIM, Ji-yoon
Daejeon 34127 (KR)**
• **OH, Seung-jin
Daejeon 34127 (KR)**
• **HO, Yo-seung
Daejeon 34022 (KR)**

(74) Representative: **Lantos, Mihaly
Danubia
Patent & Law Office LLC
Bajcsy-Zsilinszky út 16
1051 Budapest (HU)**

(56) References cited:
JP-A- H08 246 225      JP-A- S61 132 620
JP-A- 2011 200 295     KR-A- 920 012 541
KR-A- 20120 057 470    KR-B1- 101 349 262
US-A- 4 407 889        US-A1- 2006 012 072
US-A1- 2009 246 492

## Description

[Technical Field]

The present invention relates to a modified cross-section hollow fiber and fibrous assemblies using the same, and more particularly, to a modified cross-section hollow fiber with a volume control element and fibrous assemblies using the same.

[Background Art]

In general, most of synthetic fibers used in fibrous assemblies requiring functions such as thermal insulation have hollow cross-section structures and have been prepared by a representative method below.

A fiber having a hollow cross-section structure using homopolymers is used. Since heat insulation is increased by increasing dead air due to the hollow structure and an orientation difference in cross-section can be maximized in the hollow structure in cooling and oriented crystallization processes, a spontaneous crimp is expressed and thus a product having a volume can be manufactured. By the method, in order to maximize a cooling effect, productivity is deteriorated and there is a limit in crimp expression.

Another method is to use a fiber having a hollow cross-section structure using a difference in contraction between two types of polymers. As compared with the homopolymer, generally, a hollow ratio is small, but crimp expression is excellent by the difference in contraction between two types of polymers and thus it has an advantage that a volume property and elasticity are continued. In the method, further, since a product having a low viscosity is used for the crimp expression due to the difference in contraction, there is a limit in improving the hollow ratio and only complex spinning is possible.

Among the technologies, in Korean Patent Registration No. 1387465, there is provided a fiber capable of using lightweight, thermal insulation and a sweat-absorbing and quick-drying property of a multi-division hollow fiber having a modified cross-section, in which at least three hollow holes are formed at an inner side of a hollow fiber by a multi-division spinning nozzle having a modified cross-section, the outer side of the fiber is divided into the same number as the number of divided hollow holes at the inner side to form an outer slit and designed to form a modified cross-section, the multi-division hollow fiber having the modified cross-section obtained by the multi-division spinning nozzle having the modified cross-section maintains lightweight and a volume because the hollow cross-section is not easily crushed or deformed by an external force even at a high hollow ratio, and maximizes a sweat-absorbing and quick-drying property by adding a modified cross-section shape and a surface unevenness to the modified cross-section as well as unique properties of the hollow fiber.

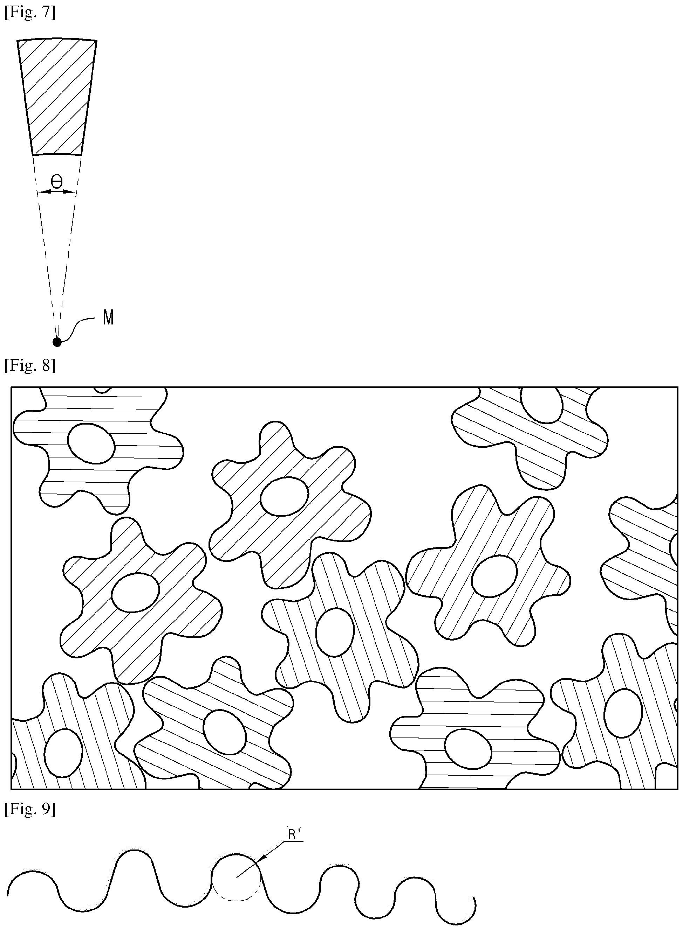

The inventors found that as a plurality of test results, the technology can form the multi-division hollow fiber, but as illustrated in FIG. 9, a protruding form of an outer slit 22 serves as an element of inhibiting bulkiness or thermal insulation formed by the hollow part by causing nonuniformity in uniformity by hindering movement of the fiber in the assemblies due to interference of the outer slit between the fibers and rather causing adhesion between the fibers to hinder formation of the space between the fibers in the assemblies, instead of serving to separate other fiber from each other in the fibrous assemblies.

Alternately, in order to improve thermal insulation, bulkiness, and the like of the fibrous assemblies, in Korean Patent Application Publication No. 2011-0069474, there is provided a nonwoven fabric having high thermal insulation prepared by a cotton carding method, in which based on a raw material, 60 to 98 wt% of a ultrafine fiber having a diameter of 4 to 15 $\mu$m consisting of a staple fiber selected from a group consisting of polyester, acrylic, polypropylene, polyethylene, urethane, rayon and acetate, 1 to 30 wt% of a hollow fiber having a diameter of 15 to 40 $\mu$m, a modified cross-section fiber, a sheath/core type fiber, a composite material fiber, or a mixed fiber thereof, and 1 to 12 wt% of a low-melting point fiber are included, the low-melting point fiber is dissolved by heating and combined with the ultrafine fiber, and the hollow fiber, the modified cross-section fiber, the sheath/core type fiber, the composite material fiber or the mixed fiber thereof.

In JP H08 246225 a modified cross-section hollow fiber has been disclosed in which the fiber is constituted by a hollow part, a shape maintaining part and volume control parts, the volume control parts may have a shape protruding away from the fiber center and have respective end parts with a round shape. Here the parameters of the shape described do not provide an esy juxtaposition of different fibers so that they can ensure the formation of dead air layers therebetween. Similar statements are ture for other pieces of prioir art including US 2009/246492.

The above technology is a general element technology to pursue lightweight and thermal insulation by using some hollow fibers and shape stability by fusing of low-melting point fibers, but there is a disadvantage in that it is impossible to ensure bulkiness and the like by the space between the fibers because the content of the ultrafine fiber is excessively high.

Accordingly, the space between the fibers can be maintained in the fibrous assemblies while ensuring dead air by ensuring shape maintenance of the hollow part and the spontaneous crimp can be formed, and thus a fiber and fibrous assemblies having complex functions such as elasticity, cushion, and sound absorption have been earnestly required.

[Disclosure]

[Technical Problem]

An object of the present invention is to provide a fiber capable of expressing various functions by ensuring an element for controlling a volume in fibrous assemblies while stably ensuring formability of a hollow part to ensure a space between fibers.

Another object of the present invention is to provide a fiber formed by variously controlling a volume control element.

Yet another object of the present invention is to provide a fiber capable of expressing various functions by expressing spontaneous crimp.

Still another object of the present invention is to provide fibrous assemblies having excellent elasticity and/or sound absorption and/or moisture discharge characteristics when bulkiness and thermal insulation are ensured.

Still yet another object of the present invention is to provide fibrous assemblies capable of consuming sound energy.

[Technical Solution]

An aspect of the present invention provides a modified cross-section hollow fiber having a fiber center, in which the fiber is constituted by a hollow part, a shape maintaining part and volume control parts, the volume control parts have a shape protruding away from the fiber center and have respective end parts with a round shape.

When the top of the end part of the volume control part is defined as a peak and a space between the volume control parts is defined as a valley, the following conditions are satisfied.

$$(1) \; -3 \leq Z \leq 4$$

$$(2) \; 0.9 \leq \frac{\sum_{i=1}^{N} Z}{} \leq 1.8$$

Herein,

Z: R-r
N: The number of volume control parts

Further, the following condition may be satisfied.

$$(3) \; (CT_{max} - R)/(CT_{min} - R) \geq 0.80$$

$$(4) \; (Ct_{max} - r)/(Ct_{min} - r) \geq 0.30$$

Herein,

T1: The largest value of a distance from a central point M to a peak 310
T2: the smallest value of a distance from the central point M to the peak 310
t1: the largest value of a distance from a central point M to a valley 330
t2: the smallest value of a distance from the central point M to the valley 330
CTmax: a circle formed by tangenting the outer peak 310 of the volume control part 300 having the second largest value of the distance between the center point M and the peak 310 based on T1
CTmin: a circle formed by tangentingof the outer peak 310 of the volume control part 300 having the second smallest value of the distance between the center point M and the peak 310 based on T2

Ctmax: a circle formed by tangenting the valley 310 from the inside of the volume control part 300 having the second largest value of the distance between the center point M and the valley 310 based on t1

Ctmin: a circle formed by tangenting the valley 310 from the inside of the volume control part 300 having the second smallest value of the distance between the center point M and the valley 310 based on t2

CTmax-R: a difference value between the center point CTmaxM of CTmax and the center point M

CTmin-R: a difference value between the center point CTminM of CTmin and the center point M

Ctmax-r: a difference value between the center point CtmaxM of Ctmax and the center point M

Ctmin-r: a difference value between the center point CtminM of Ctmin and the center point M

A shape for forming the volume control part may be prepared by a radially deployed spinneret.

A radially deployed angle θ may be 10 to 17° based on the center point M.

The number of volume control parts may be 4 to 12.

A hollow ratio of the hollow part may be 15 to 30% in a fiber cross-sectional area.

Further, the present invention provides a modified cross-section hollow fiber having staple-fiber fineness of 0,67 tex (6 De) and a fiber length of 64 mm by preparing a hollow fiber having 4, 6, or 12 volume control parts using polyester having a limiting viscosity of 0.64 and applying a crimp by a crimper after performing spinning at a spinning temperature of 285°C and a spinning velocity of 1,000 m/min and stretching at a stretching rate of 3.8.

Another aspect of the present invention provides fibrous assemblies including the fiber.

When the fibrous assemblies is prepared by a thermal bonding process, the fibrous assemblies includes 60 to 90 wt% of a modified cross-section hollow fiber and 40 to 10 wt% of a bonding material, a length of the modified cross-section hollow fiber is 51 to 64 mm, and a thickness of the fiber is 0,44-0,89 tex (4 to 8 deniers).

When the fibrous assemblies are prepared by a melt blowing process, the fibrous assemblies include 20 to 60 wt% of a modified cross-section hollow fiber and 80 to 40 wt% of a fine PP fiber, a length of the modified cross-section hollow fiber is 32 to 51 mm, and a thickness of the fiber is 0,44-0,89 tex (4 to 8 deniers).

The modified cross-section hollow fiber may include a modified cross-section fiber which applies bulkiness and sound absorption of the assemblies and simultaneously reduces a diffraction effect of sound energy to be separated between adjacent fibers in the assemblies.

Still another aspect of the present invention provides a sanitary nonwoven fabric including the modified cross-section hollow fiber.

A modified cross-section hollow fiber in the nonwoven fabric may be contained with 40 wt% or more.

The fiber may be made of two types of polymers having different intrinsic viscosities as a composite fiber, and the complex-spun fiber may have an omega (Ω) type spontaneous crimp.

The fiber may have a side-by-side hollow structure.

The fiber may be made of polyethylene terephthalate (PET), poly 1, 4-cyclohexylenedimethylene terephthalate (PCT), polypropylene (PP), nylon, and the like of two components having different viscosities.

The fiber may be constituted by polyethylene terephthalate (PET) of two components having different viscosities and have 10 to 10,000 ppm of at least one polyfunctional group selected from a group consisting of polycarboxylic acid, polyol and polyoxycarboxylic acid in the fiber.

The crimp may satisfy the following condition (5).

$$(5)\ 2.5\ \mathrm{mm} \leq \mathrm{R'} \leq 4.5\ \mathrm{mm}$$

Herein, R': Curvature radius of circular arc of crimp

Still yet another aspect of the present invention provides a nonwoven fabric for thermal insulation including the modified cross-section hollow fiber.

[Advantageous Effects]

According to the embodiment of the present invention, the modified cross-section hollow fiber can provides a fiber having a relatively high hollow ratio and a stable shape.

Further, the present invention has advantages of ensuring bulkiness in fibrous assemblies by an interference effect of the volume control parts and exhibiting high thermal insulation and heat insulation by ensuring more dead air.

Further, the present invention has features of expressing complex functionality having excellent sound absorption due to a water discharge characteristic and internal sound absorption and soundproof factors in addition to lightweight and thermal insulation together with bulkiness and elasticity by the hollow part and the spontaneous crimp structure.

Further, the fibrous assemblies including the modified cross-section hollow fiber according to the present invention has advantages of ensuring bulkiness in the fibrous assemblies by an interference effect of the volume control parts and exhibiting high heat insulation by ensuring more dead air.

[Description of Drawings]

FIGS. 1 to 6 are schematic diagrams of fiber cross-sections according to a preferred embodiment of the present invention.

FIG. 7 is a schematic diagram of a spinneret corresponding to a volume control part according to a preferred embodiment of the present invention.

FIG. 8 is a cross-sectional schematic diagram of fibrous assemblies according to a preferred embodiment of the present invention.

FIG. 9 is a schematic diagram of crimp expression according to a preferred embodiment of the present invention.

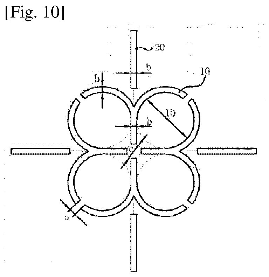

FIG. 10 is a schematic diagram of a spinneret in the related art.

[Best Mode]

Hereinafter, the present invention will be described in detail with reference to the accompanying drawings. First, it should be noted that the same elements or components in the drawings will be designated by the same reference numerals. In describing the present invention, a detailed description of publicly known functions or configurations incorporated herein will be omitted so as not to make the subject matter of the present invention unclear.

The terms representing the degree used in this specification of approximately, substantially, and the like are used as the value or a meaning close to the value when unique manufacturing and material tolerances are proposed in the aforementioned meaning, and used for preventing the disclosed content in which accurate or absolute figures are mentioned in order to help in the understanding of the present invention from being wrongly used by unscrupulous infringers.

In this specification, a fiber aggregation includes all of long fibers and staple fibers and means that one or more fibers such as cloths, knitted fabrics, fabrics, nonwoven fabrics, webs, slivers, and tows as a non-limiting example.

A modified cross-section hollow fiber according to a preferred embodiment of the present invention may be made of all materials which may be formed in a fiber shape. Preferably, polyethylene terephthalate (PET) may be used, but is not limited to it and polypropylene (PP), nylon, and the like may be used. A melt viscosity of the melt-spun PET polymer is preferably 0.60 to 0.64, and an in-out type spinning chimney capable of maximizing a cooling effect is suitable. A thickness of the fiber may be variously applied as 0,44 to 1.67 tex (4 to 15 De) and a fiber length may be 22 to 64 mm.

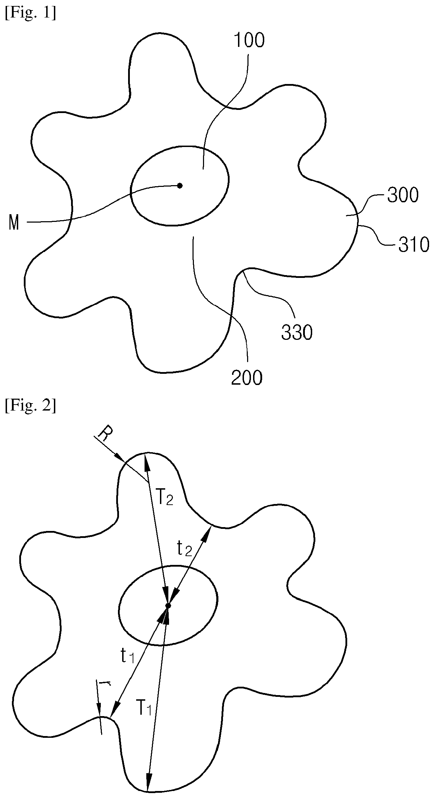

FIG. 1 is a schematic diagram of a modified cross-section hollow fiber according to a preferred embodiment of the present invention, and the fiber may be formed by a hollow part 100, a shape maintaining part 200, and a volume control part 300. A hollow ratio of the hollow part 100 may be about 15 to 30% relative to the total fiber area. In the case of more than the range, there is a problem in fiber formability, and in the case of less than the range, there is a limit in expressing hollow maintenance and various functionalities of the present invention. The shape maintaining part 200 means a fiber shape between the hollow part 100 and the volume control part 300.

The volume control part 300 may have a shape protruding in an opposite direction to the fiber center and an end part may have a rounded shape. The top of the end part of the volume control part 300 may be defined as a peak 310 and a space between the volume control parts 300 may be defined as a valley 330. In this case, a curvature radius of the peak is defined as R and a curvature radius of the valley is defined as r, and R and r values may be determined to be the same as or different from each other for each volume control part (see FIG. 2).

Further, the largest value of a distance between a center point M of the hollow part 100 and the peak 310 may be defined as T1, the smallest value of the between the center point M and the peak 310 may be defined as T2, the largest value of a distance between the center point M and the valley 330 may be defined as t1, and the smallest value of the between the center point M and the valley 330 may be defined as t2. Meanwhile, a circle formed by connecting tangents of the volume control part 300 having the second largest value of the distance between the center point M and the peak 310 based on T1 may be defined as CTmax, a circle formed by connecting tangents of the volume control part 300 having the second smallest value of the distance between the center point M and the peak 310 based on T2 may be defined as CTmin, a circle formed by connecting tangents of the volume control part 300 having the second largest value of the distance between the center point M and the valley 310 based on t1 may be defined as Ctmax, and a circle formed by connecting tangents of the volume control part 300 having the second smallest value of the distance between the center point M and the valley 310 based on t2 may be defined as Ctmin.

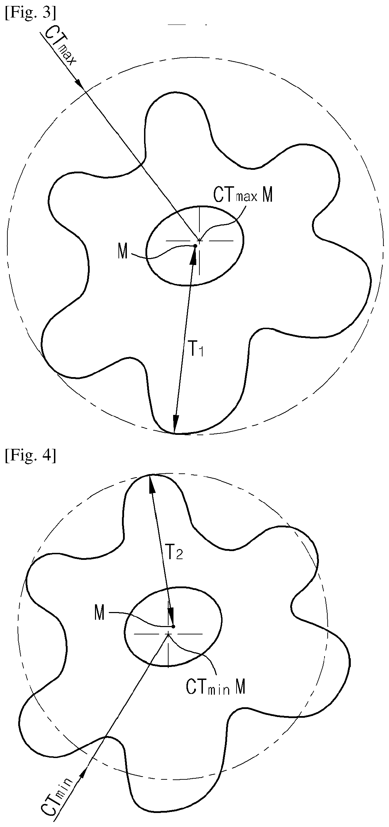

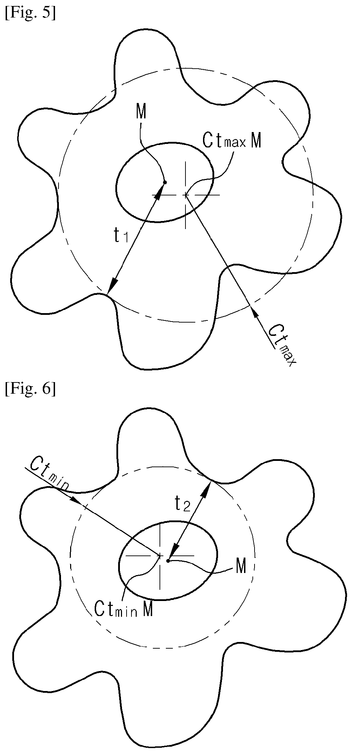

Meanwhile, when a difference value between a center point CTmaxM of CTmax and the center point M is defined as CTmax-R, a difference value between a center point CTminM of CTmin and the center point M is defined as CTmin-R, a difference value between a center point CtmaxM of Ctmax and the center point M is defined as Ctmax-r, and a difference value between a center point CtminM of Ctmin and the center point M is defined as Ctmin-r, the fiber according to the present invention may satisfy the following conditions (FIGS. 3 to 6).

When a deviation of the curvature radius R of the peak and the curvature radius r of the valley is defined as Z, the Z may be made by conditions (1) and (2) below.

$$(1)\ -3 \leq Z \leq 4$$

$$(2)\ 0.9 \leq \frac{\sum_{Z=1}^{N} Z_i}{} \leq 1.8$$

Herein,

Z: R-r

N: The number of volume control parts

As a plurality of test results of the inventors through the fiber cross-sectional shape analysis, beyond the range, a volume control part of one fiber is inserted to a valley between adjacent volume control parts of another fiber to have a structural characteristic as if gears are engaged, and it is analyzed that after insertion, the volume control part is not separated by flow and the like to have a bad effect on uniformity of the fibrous assemblies. Within the range, the volume control parts between the fibers are interfered from each other to maintain bulkiness, and even though the volume control part is inserted to the valley between the adjacent fibers, the volume control part is easily separated by flow and the like to improve the uniformity in the fibrous assemblies.

Further, in a fiber according to a preferred embodiment of the present invention, $CT_{max}$-R, $CT_{min}$-R, $Ct_{max}$-r, and $Ct_{min}$-r may satisfy the following condition.

$$(3)\ (CT_{max} - R)/(CT_{min} - R) \geq 0.80$$

$$(4)\ (Ct_{max} - r)/(Ct_{min} - r) \geq 0.30$$

Herein,

T1: The largest value of a distance from a central point M to a peak 310
T2: the smallest value of a distance from the central point M to the peak 310
t1: the largest value of a distance from a central point M to a valley 330
t2: the smallest value of a distance from the central point M to the valley 330
CTmax: a circle formed by connecting tangents of the volume control part 300 having the second largest value of the distance between the center point M and the peak 310 based on T1
CTmin: a circle formed by connecting tangents of the volume control part 300 having the second smallest value of the distance between the center point M and the peak 310 based on T2
Ctmax: a circle formed by connecting tangents of the volume control part 300 having the second largest value of the distance between the center point M and the valley 330 based on t1
Ctmin: a circle formed by connecting tangents of the volume control part 300 having the second smallest value of the distance between the center point M and the valley 330 based on t2
CTmax-R: a difference value between a center point CTmaxM of CTmax and the center point M
CTmin-R: a difference value between a center point CTminM of CTmin and the center point M
Ctmax-r: a difference value between a center point CtmaxM of Ctmax and the center point M
Ctmin-r: a difference value between a center point CtminM of Ctmin and the center point M

The conditions (3) and (4) may relate to formability of the fiber according to the embodiment of the present invention. Ideally, the value needs to be 1, but may not 1 by a rheological property of a polymer. The condition (3) may relate to formation of the volume control part, and beyond the above range, a deviation between the volume control parts is increased and a deviation of the r values may be increased to have an effect on carding in the process or bulkiness in the fibrous assemblies. The condition (4) may be interpreted as fiber morphology and have an effect on the formality of the hollow part 100 and the shape maintaining part 200. Beyond the range, the hollow formality and the shape maintenance of the fiber may be unstable.

Meanwhile, in order to form the fiber cross-section, a spinneret of the volume control part 300 may be formed in a radial shape as illustrated in FIG. 7. In this case, an angle θ may be 10 to 17° based on the center point M. As many test results of the inventors, while a hollow property is maintained within the above range, as a volume control element of a modified cross-section, a fiber cross-sectional shape capable of satisfying the above conditions for expressing the function of the member 300 is implemented.

In the cross-sectional shape of the modified cross-section hollow fiber used in the present invention, 4 to 12 volume control parts may be formed on the fiber surface.

Further, the fiber according to the embodiment of the present invention may be made of polyester as a thermoplastic resin as an non-limited example and contribute to improve bulkiness and elasticity in a staple fiber state or a nonwoven fabric shape through spontaneous crimp expression due to a difference in crystallization rate in cooling and solidifying processes.

The fiber according to the present invention may be prepared by molding fibrous assemblies including a binding material for forming only the fiber or a binding structure between fibers according to the present invention in a nonwoven fabric form through a needle punching process, a thermal bonding process, or a melt blowing process.

In the fibrous assemblies applying the modified cross-section hollow fiber according to the present invention, as a staple fiber shape of a binding material generally used for binding between the fibers, in the thermal bonding process, a sheath-core modified low-melting point PET staple fiber may be used and in the melt blowing process, a fine PP fiber may be used.

The material prepared in the thermal bonding process is constituted by a composition including 60 to 90 wt% of the modified cross-section hollow fiber and 40 to 10 wt% of the bonding material, and herein, a length of the modified cross-section hollow fiber may have 51 to 64 mm and the thickness (fineness) of the fiber may be 0,44 to 0,89 tex (4 to 8 deniers). When the length of the modified cross-section hollow fiber is less than 51 mm in the thermal bonding process, a gap between the fibers is increased, it is difficult to form a matrix structure, and it is difficult to form and produce the fibrous assemblies. Further, a result of deteriorating sound absorption and sound performance due to excessive porosity may be caused.

A composition weight ratio of the modified cross-section hollow fiber and the bonding material is preferably 6:4 to 9:1. Herein, when the content of the modified cross-section hollow fiber is less than 60 wt%, the surface area of the fiber is reduced and thus physical properties cannot be implemented, and particularly, since the content of low-melting point PET used in the thermal bonding process is relatively increased, bulkiness having large porosity is not maintained and the fibrous assemblies are hardened. On the contrary, when the content of the modified cross-section hollow fiber is more than 90 wt%, relatively, the content of a binder fiber, that is, the bonding material is less than 10% and thus sufficient bonding force between the fibers is not maintained, and as a result, it is difficult to form the assemblies in any shape.

The material prepared in the melt blowing process is constituted by a composition including 20 to 60 wt% of the modified cross-section hollow fiber and 80 to 40 wt% of a fine PP fiber, and herein, a length of the modified cross-section hollow fiber may have 32 to 51 mm and the thickness (fineness) of the fiber may be 0,44 to 0,89 tex (4 to 8 deniers). When the length is more than 51 mm, in a blowing process by air after opening, an ununiform web is formed due to fiber entanglement. As a result, according to a subsequent process applied to a sound absorbing material and a filler, it is required to select a suitable fiber length in a range of 32 to 64 mm.

A nonwoven fabric prepared by the thermal bonding process or the melt blowing process may ensure a dead air layer in the space between fibers made by contacting the fiber adjacent to the volume control part 300 of the modified cross-section fiber. The dead air layer may ensure a wider dead air layer so that a space between the fibers may be formed by contacting the peak 310 of the fiber and the peak 310 of the adjacent fiber.

Thermal insulation by the dead air layer is used to store a large amount of air in pores between the fibers. Dead air ensured by the dead air layer is supported by the fiber as immobile air to have small mobility and may have thermal insulation due to a function of blocking heat without transmitting the heat.

The volume control parts 300 are limited to a predetermined range and thus the volume control part 300 between the fibers is not easily inserted to the valley 330 of the adjacent fibers and even though the volume control part 300 is inserted, the volume control part 300 may be easily separated and thus it is advantageous in ensuring the dead air layer.

The thermal insulation of a heat retaining nonwoven fabric including a modified cross-section hollow fiber according to an embodiment of the present invention is improved by ensuring dead air layers more than the heat retaining nonwoven fabric including a circular cross-section fiber or a circular cross-section hollow fiber in Comparative Example.

The circular structure has excellent density compared with a modified structure and an arrangement between adjacent fibers is closer, and thus it is difficult to widely ensure the dead air layer.

Further, the structure of the hollow fiber has a hollow layer 100 for each fiber to more ensure the dead air layer. Air is present in the hollow layer 100 to maintain thermal insulation by using low thermal conductivity of the air.

In a nonwoven fabric including the modified cross-section hollow fiber according to an embodiment of the present invention, density is relatively deteriorated by the modified cross-section structure and the volume control element to ensure more dead air layers and simultaneously more improve thermal insulation by holding the dead air layer through the hollow layer.

A method of measuring the thermal insulation may use KS K 0560 (2011 constant temperature method).

In the constant temperature method which is one of the KS K 0560 methods, preliminary tension is applied to a specimen by using a thermal insulation retaining tester having appropriate performance and then attached to a constant temperature heater. A heat loss emitted by transmitting the specimen after 2 hours from the time when an amount of the heat released to outer air at a low temperature becomes constant and then a surface temperature of the heater has a constant value is calculated. The value and a heat loss emitted at the same temperature difference while there is no specimen and at the same time are calculated to measure thermal insulation due to a difference between the two values.

A thermal insulation ratio may be calculated by the following Equation based on the KS K 0560.

The thermal insulation ratio (%) is $(1-\alpha 2/\alpha 1)\times 100$, and herein, $\alpha 1$ is a heat dissipation amount ($cal/cm^2/sec$ or w/hr) when there is no specimen in the heater and $\alpha 2$ is a heat dissipation amount when the specimen is attached to the heater.

Meanwhile, in the present invention, even as the sound absorbing material, the function is expressed, and the sound absorption means that an object absorbs the sound. As the sound absorbing material, a plurality of fiber materials is used, and in energy of a sound input to a fiber material, a part is reflected from the surface, a part is transmitted, and the remaining part is absorbed in the material. The sound absorption in the material occurs due to friction, viscous resistance, or vibration of a fibril in the case of a porous material, membrane vibration in the case of a thin plate or cloth, and a loss of sound energy by resonance in the case of a narrow jar.

The sound absorption means that when the sound is projected to one side of the material and observed only at the side, a non-reflected sound is absorbed and transmitted to the material, and apparently, the sound is absorbed in the material, and an energy ratio of the non-reflected sound to energy of the input sound is referred to as a sound absorption ratio. The sound absorption ratio varies according to a frequency of the sound, an incident angle, a thickness of the material, an installation method, a situation on the back side, and the like. A sound absorbing material having various sound absorption ratios is used for improving a sound effect in the interior or lowering a noise level.

Further, the sound has a characteristic of transmitting the sound by a diffraction effect as the energy. Due to the characteristic, even in a space installed with the sound absorbing material, the sound may be propagated to the outside. Accordingly, in the fiber according to the present invention, a function capable of suppressing a sound transmission phenomenon by the diffraction effect as well as the sound absorption will be further proposed.

In the fiber according to the present invention, the volume control part ensures the bulkiness by a physical interference in the fibrous assemblies as described above to further ensure the space therebetween, thereby improving the sound absorption through vibration of the fiber, securing of the relative thickness, and the like.

Further, the volume control part may have a characteristic that sound energy propagated through the diffraction effect by enlarging a specific surface area compared with a circular cross-section is consumed while moving along the volume control part according to the present invention to be reduced. As a result, the fiber and the fibrous assemblies according to the present invention may achieve sound insulation and sound blocking effects.

Meanwhile, in the fibrous assemblies according to the embodiment of the present invention, fine pores in the assemblies are generated by the volume control element to express a water transition characteristic by a capillary phenomenon. In the concept of water discharge, it is important to remove an absolute amount of water retained in the assemblies from the assemblies, but the water rapidly moves to another constitute element in the assemblies to express a water discharge function. For example, when the fibrous assemblies according to the present invention is used for a surface sheet of a diaper or a sanitary pad, a water element generated in the human body is rapidly absorbed through a capillary phenomenon and may rapidly move to an absorbing layer formed on a back surface thereof. In this case, the generated water element rapidly moves to improve an absorption rate and

simultaneously, water rapidly moves to the absorbing layer from the surface contacting the skin surface again to express comfortability.

Meanwhile, the modified cross-section hollow fiber according to the present invention may be prepared by complex spinning. In this case, the modified cross-section hollow composite fiber may be made of all materials which may be formed in a fibrous shape. Preferably, polyethylene terephthalate (PET) having a different viscosity may be used, but is not limited and polypropylene (PP), nylon, poly 1, 4-cyclohexylenedimethylene terephthalate (PCT), and the like may be used by complex spinning.

In two types of polymers having different intrinsic viscosities, a polymer having a relative high intrinsic viscosity may be defined as a first polymer and a polymer having a relative low intrinsic viscosity may be defined as a second polymer.

The modified cross-section hollow composite fiber according to the embodiment of the present invention includes all intrinsic viscosities which may be formed in a fibrous shape. Preferably, the first polymer may have an intrinsic viscosity of 0.60 to 0.80 and the second polymer may use a polymer having an intrinsic viscosity of 0.50 to 0.64.

Further, the fiber according to the embodiment of the present invention may be made of two types of polyesters having different intrinsic viscosities as an non-limited example and contribute to improve bulkiness and elasticity in a staple fiber state or a nonwoven fabric shape through spontaneous crimp expression due to a difference in intrinsic viscosity. In this case, the spontaneous crimp shape may be an omega shape (that is, similar to a Greek letter ($\Omega$)) and the top may be formed in a rounded shape.

The omega-shaped crimp has a spontaneous crimp shape expressed in a side-by-side type composite fiber and a omega-shaped fiber crimp exhibits excellent bulkiness and restoration force after compression as compared with a zigzag-shaped fiber crimp which is given artificially.

More preferably, PET polymers having an intrinsic viscosity of 0.64 and an intrinsic viscosity of 0.55 may be complex-spun. When the first and second polymers have intrinsic viscosities of 0.50 or less, the cross-section is closer to a circle, and thus it is not desirable to implement a modified cross-section and the hollow formation is also difficult.

Meanwhile, the modified cross-section hollow composite fiber may be a modified cross-section hollow composite fiber made of polyethylene terephthalate (PET), poly 1, 4-cyclohexylenedimethylene terephthalate (PCT), polypropylene (PP), nylon, and the like of two components having different viscosities. Further, the modified cross-section hollow composite fiber may be a modified cross-section hollow composite fiber which is constituted by two polyethylene terephthalates (PET) having different viscosities and has 10 to 10,000 ppm of at least one polyfunctional group selected from a group consisting of polycarboxylic acids, polyols, and polyoxycarboxylic acids in the fiber.

In the composite fiber according to the embodiment of the present invention, a spontaneous crimp may be expressed in addition to the hollow fiber as one of the embodiments, and the crimp may have an omega shape and the top may be formed in a rounded shape. A curvature radius of the crimp may be defined as R' and may vary according to fineness of the spun fiber, but may basically satisfy the following condition (5) (see FIG. 9).

$$(5)\ 2.5\ \text{mm} \leq \text{R'} \leq 4.5\ \text{mm}$$

Herein, R': Curvature radius of circular arc of crimp

Hereinafter, the present invention will be described by the following Examples.

Examples 1 to 3

Fibers having 4, 6, and 12 volume control parts were prepared by using polyester having a limiting viscosity of 0.64. At a spinning temperature of 285°C, a fiber having staple-fiber fineness of 0,66 tex (6 De) and a fiber length of 64 mm was prepared by applying a crimp by a crimper after performing spinning at a spinning velocity of 1,000 m/min and then stretching at a stretching rate of 3.8.

Comparative Examples 1 to 3

Comparative Examples 1 to 3 are the same as Example 1, but a circular cross-section fiber, a circular hollow cross-section fiber, and a complex-spun circular hollow cross-section fiber with polyester limiting viscosities of 0.64 and 0.50 were prepared.

Examples 4 to 6

Fibers having 4, 6, and 12 volume control parts were prepared by using polyester having a limiting viscosity of 0.64. A fiber having staple-fiber fineness of 0.58 tex (5.2 De) and 13.9/24 tex/filament (125/24De/fil.) was prepared by applying a crimp by a crimper after performing spinning at a spinning temperature of 285°C and a spinning velocity of 1,000 m/min and then stretching at a stretching rate of 3.8 and then warp knitted matters were prepared by using the fibers, respectively.

Example 7

A fiber having 6 volume control parts was prepared by using polyester having a limiting viscosity of 0.64. A fiber having staple-fiber fineness of 0.67 tex (6 De)and a fiber length of 64 mm was prepared by applying a crimp by a crimper after performing spinning at a spinning temperature of 285°C and a spinning velocity of 1,000 m/min and then stretching at a stretching rate of 3.8. The fiber was opened and then prepared in a web form.

Example 8

A fiber having 6 volume control parts was prepared by using polyester having a limiting viscosity of 0.64. A fiber having staple-fiber fineness of 0.67 tex (6 De) and a fiber length of 64 mm was prepared by applying a crimp by a crimper after performing spinning at a spinning temperature of 285°C and a spinning velocity of 1,000 m/min and then stretching at a stretching rate of 3.8. The fiber was used and combined with a polyester-based low-melting point yarn to be 25 wt% to prepare a nonwoven fabric by needle punching. The nonwoven fabric had a size of 840*840 (mm*mm) and a weight of about 330 g.

Example 9

A fiber having 6 volume control parts was prepared by using polyester having a limiting viscosity of 0.64. A fiber having staple-fiber fineness of 0,67 tex (6 De) and a fiber length of 64 mm was prepared by applying a crimp by a crimper after performing spinning at a spinning temperature of 285°C and a spinning velocity of 1,000 m/min and then stretching at a stretching rate of 3.8. The fiber was used and combined with a polyester-based low-melting point yarn to be 25 wt% to prepare a nonwoven fabric by needle punching. The nonwoven fabric had a size of 840*840 (mm*mm) and a weight of about 380 g.

Example 10

A fiber having 6 volume control parts was prepared by using polyester having a limiting viscosity of 0.64. A fiber having staple-fiber fineness of 0.67 tex (6 De) and a fiber length of 64 mm was prepared by applying a crimp by a crimper after performing spinning at a spinning temperature of 285°C and a spinning velocity of 1,000 m/min and then stretching at a stretching rate of 3.8. The fiber was used and combined with a polyester-based low-melting point yarn to be 25 wt% to prepare a nonwoven fabric by needle punching. The nonwoven fabric had a size of 840*840 (mm*mm) and a weight of about 440 g.

Example 11

A fiber having 6 volume control parts was prepared by using polyester having a limiting viscosity of 0.64. A fiber having staple-fiber fineness of 0.67 tex (6 De)and a fiber length of 64 mm was prepared by applying a crimp by a crimper after performing spinning at a spinning temperature of 285°C and a spinning velocity of 1,000 m/min and then stretching at a stretching rate of 3.8. The fiber was used and combined with a polypropylene meltblown yarn to be 55 wt% to prepare a nonwoven fabric. The nonwoven fabric had a size of 840*840 (mm*mm), a weight of about 240g, and a thickness of about 20 mm.

Examples 12 and 13

A fiber having 6 volume control parts was prepared by using polyester having a limiting viscosity of 0.64. A fiber having staple-fiber fineness of 4 De and 6 De and a fiber length of 51mm was prepared by applying a crimp by a crimper after performing spinning at a spinning temperature of 285°C and a spinning velocity of 1,000 m/min and then stretching at a stretching rate of 3.8.

Examples 14 and 15

The fiber of Example 12 was used and combined with a polyester-based low-melting point yarn to be 25 wt% to prepare a nonwoven fabric by needle punching. The nonwoven fabric had a size of 840*840 (mm*mm) and a weight of about 400 g and 700 g.

Examples 16 and 17

The fiber of Example 13 was used and combined with a polyester-based low-melting point yarn to be 25 wt% to prepare a nonwoven fabric by needle punching. The nonwoven fabric had a size of 840*840 (mm*mm) and a weight of about 400 g and 700 g.

Comparative Examples 4 to 6

Comparative Examples 4 to 6 are the same as Example 4, but a circular cross-section fiber (Comparative

**EP 3 196 342 B1**

Example 4), a circular hollow fiber (Comparative Example 5), and a ⬡ -modified cross-section fiber (Comparative Example 6) were prepared. In this case, a fiber having staple-fiber fineness of 0.28 tex (2.5 De) and 1.39/24 tex/filament (125/24De/fil.) was prepared and then a warp knitted matter was prepared by using the fibers, respectively.

Comparative Examples 7 to 9

Comparative Examples 7 to 9 are the same as Example 7, but a circular cross-section fiber, a circular hollow fiber, and a complex-spun circular hollow cross-section fiber with polyester intrinsic viscosities of 0.64 and 0.50 were prepared. In this case, a fiber having staple-fiber fineness of 0.67 tex (6 De) and a fiber length of about 64 mm was prepared and then a web form was prepared after opening by using the fibers.

Comparative Examples 10 and 11

Comparative Examples 10 and 11 were the same as Example 8, but a circular cross-section fiber and a

⬡ -modified 8-leaf cross-section fiber were prepared. In this case, a fiber having staple-fiber fineness of 0.67 tex (6 De) and a fiber length of about 64 mm was prepared. The fiber was used and combined with a polyester-based low-melting point yarn to be 25 wt% to prepare a nonwoven fabric by needle punching. The nonwoven fabric had a size of 840*840 (mm*mm) and a weight of about 330 g.

Comparative Examples 12 and 13

Comparative Examples 12 and 13 were the same as Example 9, but a circular cross-section fiber and a

⬡ -modified 8-leaf cross-section fiber were prepared. In this case, a fiber having staple-fiber fineness of 0,67 tex (6 De) and a fiber length of about 64 mm was prepared. The fiber was used and combined with a polyester-based low-melting point yarn to be 25 wt% to prepare a nonwoven fabric by needle punching. The nonwoven fabric had a size of 840*840 (mm*mm) and a weight of about 380 g.

Comparative Examples 14 and 15

Comparative Examples 14 and 15 were the same as Example 10, but a circular cross-section fiber and a

⬡ -modified 8-leaf cross-section fiber were prepared. In this case, a fiber having staple-fiber fineness of 0,67 tex (6 De) and a fiber length of about 64 mm was prepared. The fiber was used and combined with a polyester-based low-melting point yarn to be 25 wt% to prepare a nonwoven fabric by needle punching. The nonwoven fabric had a size of 840*840 (mm*mm) and a weight of about 440 g.

Comparative Example 16

A fiber having staple-fiber fineness of 0,67 tex (6 De) and a fiber length of 64 mm was prepared by applying a crimp by a crimper after spinning a hollow cross-section fiber at a spinning temperature of 285°C and a spinning velocity of 1,000 m/min by using polyester having a limiting viscosity of 0.64 and then stretching the spun hollow cross-section fiber at a stretching rate of 3.8. The fiber was used and combined with a polypropylene meltblown yarn to be 55 wt% to prepare a nonwoven fabric. The nonwoven fabric had a size of 840*840 (mm*mm), a weight of about 240 g, and a thickness of about 20 mm.

Comparative Examples 17 and 18

Under the same condition as Examples 12 and 13, a circular cross-section fiber of 0.44 tex (4 De) and a hollow composite staple fiber of 0.67 text (6 De) were prepared.

Comparative Examples 19 and 20

The fiber of Examples 18 and 19 was used and combined with a polyester-based low-melting point yarn to be 25 wt% to prepare a nonwoven fabric by needle punching. The nonwoven fabric had a size of 840*840 (mm*mm) and a weight of about 400 g and 700 g.

In Examples and Comparative Examples below, measurement of physical properties was performed as follows.

* Bulkiness of composite fiber

A. Test method

- A prepared sample was quantified with $20\pm2$g.
- The sample was opened for 1 minute by using an opening device.
- The opened sample was put in a measuring beaker and downed two times (4 cm) to the end part to be uniformly filled.
- A pressure plate was positioned on the top of a container and then an electronic balance was set to "0".
- A weight of the balance was recorded while decreased by a unit of 1 cm from an initial point of 10 cm to a point of 4 cm.
- The weight was recorded while increased up to a point of 10 cm again.

(Scale moving speed of 2 sec/cm)

B. Bulkiness

- Initial bulky: A bulky characteristic of fiber, a value during 10 cm compression
- Compressive bulky: Repulsion characteristic of fiber, (compression value of 10 to 5 cm + value of 4 cm) / 2
- Restorative bulky: Elastic restorative characteristic of fiber, (restoration value of 10 to 5 cm + value of 4 cm) / 2

* Hollow ratio

A hollow ratio of the fiber was calculated by a ratio of an area occupied by a hollow part to the entire area and measured by an area ratio occupied by a hollow part to an area of an inner circle contacting a pinion in the case of a hollow fiber having volume control parts.

* Sound characteristics

A. Measurement of sound absorption rate by reverberation method

A sound absorption rate was measured by using equipment equivalent to ISO 354 (KS F 2805: method of measuring sound absorption rate in reverberation room). A size of the specimen was set to 1.0 m x 1.2 m, a reverberation time was the time when the sound pressure was reduced to 20 dB compared with initial sound pressure, and a 1/3 Octave band sound source was used as a sound source. At a frequency range of 0.4 to 10 kHz, a sound absorption rate was measured.

* Evaluation test of water absorption rate of fiber

A. Application range

A feather touch fiber applied to advanced padding was washed and then in order to find a dehydration effect, a water absorption rate test was evaluated.

B. Outline of method

An absorption rate was evaluated by comparing weights before immersing in distilled water and after dehydration after opening the fiber.

C. Appliances and devices

- Opening appliance
- Two sample bags (100% nylon knitted fabric: warp (92 patterns/inch), weft (60 patterns/inch), 12 cm x 12 cm)
- Stapler, fine pin, steel bar
- Water bath

D. Test Method

- 30 g of a prepared fiber was opened with air for 1 min by an opening appliance.
- The opened fiber was quantified by 10 g twice.
- The opened fiber was put in each of two sample bags, the bags were sealed by a stapler, and then a weight Ms was measured.
- The two sample bags were hung on a steel bar by using a fine pin.
- The two sample bags were immersed in a water bath with distilled water at $20\pm2°C$ for 1 hr.
- The two sample bags were dehydrated for 30 minutes.
- A weight Mf of the dehydrated sample bags after immersing was measured.

E. Measurement

- Samples before and after absorption

A weight Ms before immersing the sealed sample bag was measured.
A weight Mf after immersing for 1 hr and dehydrating for 30 minutes was measured.
- Calculation method

$$\text{Water absorption rate } (\%) = (Mf-Ms)/Ms \times 100$$

[Table 1]

| | | Example | | | Comparative Example | | |
|---|---|---|---|---|---|---|---|
| | | 1 | 2 | 3 | 1 | 2 | 3 |
| Cross-sectional shape | | 4 volume control parts, hollow | 6 volume control parts, hollow | 12 volume control parts, hollow | Circle | Circular hollow | Circular hollow |
| Hollow ratio | | 19 | 21 | 18 | - | 22 | 6 |
| Bul ky | Initial | 310 | 390 | 380 | 20 | 47 | 280 |
| | Compr ession | 11050 | 11900 | 11000 | 6500 | 8800 | 9000 |
| | Restor ation | 4600 | 4900 | 4800 | 2500 | 3400 | 3900 |

Like Table 1 above, it was tested that the fiber according to the present invention had an excellent bulkiness due to an interference effect of the volume control parts, and as illustrated in FIG. 8, it was illustrated that in the fibers according to the present invention in the fibrous assemblies, the volume control part was interfered with the volume control part of the adjacent fiber to ensure a space and improve the bulkiness.

[Table 2]

| Classification | Example 4(Hz) | Example 5(Hz) | Example 6(Hz) | Comparative Example4 (Hz) | Comparative Example 5(Hz) | Comparative Example 6(Hz) |
|---|---|---|---|---|---|---|
| 0.4k | 0.1390 | 0.1120 | 0.1230 | 0.0721 | 0.1170 | 0.0734 |
| 0.5k | 0.0685 | 0.0633 | 0.0656 | 0.0656 | 0.0889 | 0.0622 |
| 0.63k | 0.0598 | 0.0594 | 0.0596 | 0.0347 | 0.0683 | 0.0231 |
| 0.8k | 0.1070 | 0.1050 | 0.1055 | 0.0617 | 0.1080 | 0.0634 |
| 1k | 0.1320 | 0.1300 | 0.1313 | 0.0800 | 0.1370 | 0.0747 |
| 1.25k | 0.1970 | 0.1960 | 0.1961 | 0.1050 | 0.1940 | 0.1030 |
| 1.6k | 0.1960 | 0.2010 | 0.1980 | 0.1340 | 0.2080 | 0.1260 |
| 2k | 0.1750 | 0.1690 | 0.1720 | 0.1100 | 0.1870 | 0.1050 |
| 2.5k | 0.1560 | 0.1450 | 0.1510 | 0.0987 | 0.1520 | 0.0954 |
| 3.15k | 0.1860 | 0.1710 | 0.1810 | 0.1000 | 0.1950 | 0.0958 |
| 4k | 0.1630 | 0.1490 | 0.1610 | 0.0782 | 0.2000 | 0.0935 |
| 5k | 0.1160 | 0.0931 | 0.1060 | 0.0832 | 0.1450 | 0.0855 |
| 6.3k | 0.0529 | 0.0547 | 0.0528 | 0.0454 | 0.0934 | 0.0128 |
| 8k | 0.1560 | 0.0853 | 0.1460 | 0.1170 | 0.2220 | 0.0488 |
| 10k | 0.0872 | 0.1440 | 0.1170 | 0.0637 | 0.2010 | 0.0034 |

[Table 3]

| Classification | Example 7 (Hz) | Comparative Example 7 (Hz) | Comparative Example 8 (Hz) | Comparative Example 9 (Hz) |
|---|---|---|---|---|
| 0.4k | 0.22 | 0.20 | 0.20 | 0.20 |
| 0.5k | 0.41 | 0.37 | 0.36 | 0.35 |
| 0.63k | 0.47 | 0.43 | 0.40 | 0.39 |
| 0.8k | 0.51 | 0.47 | 0.45 | 0.42 |
| 1k | 0.59 | 0.55 | 0.54 | 0.50 |
| 1.25k | 0.64 | 0.60 | 0.59 | 0.54 |
| 1.6k | 0.64 | 0.61 | 0.62 | 0.55 |
| 2k | 0.64 | 0.61 | 0.60 | 0.56 |
| 2.5k | 0.65 | 0.61 | 0.58 | 0.55 |
| 3.15k | 0.61 | 0.57 | 0.54 | 0.52 |
| 4k | 0.57 | 0.53 | 0.52 | 0.50 |
| 5k | 0.59 | 0.52 | 0.52 | 0.51 |

[Table 4]

| Classification | Example 8(Hz) | Comparative Example 10(Hz) | Comparative Example 11(Hz) |
|---|---|---|---|
| 0.4k | 0.18 | 0.18 | 0.15 |
| 0.5k | 0.23 | 0.23 | 0.20 |
| 0.63k | 0.22 | 0.22 | 0.20 |

(continued)

| Classification | Example 8(Hz) | Comparative Example 10(Hz) | Comparative Example 11(Hz) |
|---|---|---|---|
| 0.8k | 0.24 | 0.23 | 0.21 |
| 1k | 0.23 | 0.22 | 0.20 |
| 1.25k | 0.22 | 0.21 | 0.20 |
| 1.6k | 0.27 | 0.25 | 0.25 |
| 2k | 0.34 | 0.31 | 0.30 |
| 2.5k | 0.40 | 0.36 | 0.36 |
| 3.15k | 0.40 | 0.37 | 0.36 |
| 4k | 0.39 | 0.38 | 0.36 |
| 5k | 0.46 | 0.46 | 0.43 |
| 6.3k | 0.53 | 0.52 | 0.50 |
| 8k | 0.57 | 0.55 | 0.55 |
| 10k | 0.62 | 0.62 | 0.66 |

[Table 5]

| | Example 9(Hz) | Comparative Example 12(Hz) | Comparative Example 13(Hz) |
|---|---|---|---|
| 0.4k | 0.16 | 0.18 | 0.15 |
| 0.5k | 0.21 | 0.23 | 0.21 |
| 0.63k | 0.22 | 0.22 | 0.21 |
| 0.8k | 0.24 | 0.21 | 0.21 |
| 1k | 0.24 | 0.20 | 0.20 |
| 1.25k | 0.23 | 0.19 | 0.19 |
| 1.6k | 0.29 | 0.24 | 0.24 |
| 2k | 0.36 | 0.31 | 0.30 |
| 2.5k | 0.43 | 0.37 | 0.37 |
| 3.15k | 0.42 | 0.37 | 0.37 |
| 4k | 0.41 | 0.36 | 0.36 |
| 5k | 0.47 | 0.43 | 0.44 |
| 6.3k | 0.54 | 0.50 | 0.51 |
| 8k | 0.59 | 0.56 | 0.53 |
| 10k | 0.71 | 0.61 | 0.52 |

[Table 6]

| | Example 10(Hz) | Comparative Example 14(Hz) | Comparative Example 15(Hz) |
|---|---|---|---|
| 0.4k | 0.11 | 0.15 | 0.18 |
| 0.5k | 0.21 | 0.20 | 0.22 |
| 0.63k | 0.23 | 0.21 | 0.21 |
| 0.8k | 0.25 | 0.22 | 0.22 |

(continued)

|  | Example 10(Hz) | Comparative Example 14(Hz) | Comparative Example 15(Hz) |
|---|---|---|---|
| 1k | 0.25 | 0.22 | 0.20 |
| 1.25k | 0.24 | 0.21 | 0.19 |
| 1.6k | 0.30 | 0.26 | 0.24 |
| 2k | 0.38 | 0.33 | 0.32 |
| 2.5k | 0.45 | 0.39 | 0.40 |
| 3.15k | 0.45 | 0.39 | 0.39 |
| 4k | 0.44 | 0.37 | 0.37 |
| 5k | 0.52 | 0.43 | 0.45 |
| 6.3k | 0.62 | 0.51 | 0.53 |
| 8k | 0.66 | 0.55 | 0.58 |
| 10k | 0.69 | 0.60 | 0.65 |

[Table 7]

|  | Example 11(Hz) | Comparative Example 16(Hz) |
|---|---|---|
| 0.4k | 0.25 | 0.20 |
| 0.5k | 0.46 | 0.38 |
| 0.63k | 0.58 | 0.46 |
| 0.8k | 0.72 | 0.58 |
| 1k | 0.94 | 0.77 |
| 1.25k | 1.12 | 0.93 |
| 1.6k | 1.14 | 0.99 |
| 2k | 1.03 | 0.96 |
| 2.5k | 1.02 | 0.99 |
| 3.15k | 0.98 | 0.96 |
| 4k | 0.89 | 0.87 |
| 5k | 0.92 | 0.86 |
| 6.3k | 0.93 | 0.84 |
| 8k | 0.94 | 0.80 |
| 10k | 0.95 | 0.74 |

[Table 8]

| Classification | Example 14 | Example 15 | Comparative Example 19 | Example 16 | Example 17 | Comparative Example 20 |
|---|---|---|---|---|---|---|
| 400 | 0.17 | 0.14 | 0.17 | 0.17 | 0.19 | 0.19 |
| 500 | 0.18 | 0.14 | 0.13 | 0.19 | 0.2 | 0.19 |
| 630 | 0.15 | 0.11 | 0.09 | 0.18 | 0.19 | 0.17 |
| 800 | 0.17 | 0.13 | 0.11 | 0.23 | 0.23 | 0.21 |

(continued)

| Classification | Example 14 | Example 15 | Comparative Example 19 | Example 16 | Example 17 | Comparative Example 20 |
|---|---|---|---|---|---|---|
| 1k | 0.23 | 0.19 | 0.17 | 0.3 | 0.3 | 0.27 |
| 1.25k | 0.32 | 0.25 | 0.22 | 0.4 | 0.38 | 0.36 |
| 1.6k | 0.42 | 0.3 | 0.28 | 0.52 | 0.48 | 0.46 |
| 2k | 0.47 | 0.34 | 0.3 | 0.6 | 0.54 | 0.53 |
| 2.5k | 0.51 | 0.37 | 0.34 | 0.64 | 0.61 | 0.57 |
| 3.15k | 0.52 | 0.38 | 0.37 | 0.64 | 0.59 | 0.56 |
| 4k | 0.55 | 0.41 | 0.42 | 0.65 | 0.59 | 0.57 |
| 5k | 0.55 | 0.49 | 0.44 | 0.67 | 0.62 | 0.58 |
| 6.3k | 0.59 | 0.5 | 0.48 | 0.68 | 0.65 | 0.61 |
| 8k | 0.65 | 0.56 | 0.56 | 0.68 | 0.65 | 0.64 |
| 10k | 0.54 | 0.5 | 0.44 | 0.48 | 0.47 | 0.48 |

In Tables 2 to 8 above, by comparing sound absorption rates in Examples and Comparative Examples of the fibrous assemblies formed of the fiber according to the present invention, the result that the fiber assemblies according to the present invention had excellent sound absorption was derived.

Further, like Table 9 below, it was tested that the fiber according to the present invention had an excellent water discharge characteristic (low water absorption rate) due to an interference effect of the volume control parts, and as illustrated in FIG. 8, it was illustrated that in the fibers according to the present invention in the fibrous assemblies, the volume control part was interfered with the volume control part of the adjacent fiber to ensure a space and improve a water discharge property.

[Table 9]

| | Ms(g) | Mf(g) | Absorption rate (%) | Average absorption rate (%) |
|---|---|---|---|---|
| Example 12 | 11.66 | 16.51 | 41.60 | 41.1 |
| | 11.68 | 16.42 | 40.58 | |
| Example 13 | 11.68 | 19.41 | 66.18 | 67.7 |
| | 11.66 | 19.74 | 69.30 | |
| Comparative Example 18 | 11.37 | 100.03 | 779.77 | 686.3 |
| | 11.37 | 78.77 | 592.79 | |
| Comparative Example 19 | 11.60 | 26.76 | 130.69 | 130.0 |
| | 11.52 | 26.41 | 129.25 | |

Example 18

A fiber was prepared by a spinneret having 6 volume control parts by using polyester having intrinsic viscosities of 0.64 and 0.50. A fiber having staple-fiber fineness of 0.67 tex (6 De) and a fiber length of 64 mm was prepared by applying a crimp by a crimper after performing spinning at a spinning temperature of 285°C and a spinning velocity of 1,000 m/min and then stretching at a stretching rate of 3.8.

Example 19

A fiber was prepared by a spinneret having 6 volume control parts by using polyester having intrinsic viscosities of 0.64 and 0.55 under the same condition as Example 18.

Example 20

A fiber was prepared by a spinneret having 6 volume control parts by using polyester containing 900 ppm of polyfunctional groups and having intrinsic viscosities of 0.64 and 0.60 under the same condition as Example 18.

Comparative Example 21

A fiber was prepared by a spinneret having 6 volume control parts by using polyester having an intrinsic viscosity of 0.64. A fiber having staple-fiber fineness of 0.78 tex (7 De) and a fiber length of 64 mm was prepared by applying a crimp by a crimper after performing spinning at a spinning temperature of 285°C and a spinning velocity of 1,000 m/min and then stretching at a stretching rate of 3.8.

Comparative Examples 22 to 25

A fiber was prepared under the same condition as Example 18, and by applying a spinneret to a circular hollow cross-section and using

- polyester having intrinsic viscosities of 0.64 and 0.46
- polyester having intrinsic viscosities of 0.64 and 0.50
- polyester having intrinsic viscosities of 0.64 and 0.55
- polyester containing 900 ppm of polyfunctional groups and having intrinsic viscosities of 0.64 and 0.60, a circular hollow cross-section fiber was prepared.

Comparative Example 26

Comparative Example 26 was the same as Comparative Example 16, and a nonwoven fabric was prepared with the circular cross-section fiber.

[Table 10]

| | | Example | | | Comparative Example | | | | |
|---|---|---|---|---|---|---|---|---|---|
| | | 18 | 19 | 20 | 21 | 22 | 23 | 24 | 25 |
| Spinneret type | | 6 volume control parts, hollow | | | 6 volume control parts, hollow | Hollow circle | | | |
| High/low PET intrinsic viscosity | | 0.64/0.50 | 0.64/0.55 | | 0.64+polyfunctional group/ 0.60 | 0.64 | 0.64/0.46 | 0.64/0.50 | 0.64/0.55 | 0.64+polyfunctional group/ 0.60 |
| Hollow ratio | | | 11 | 17 | 25 | 21 | 5 | 12 | 18 | 24 |
| Crimp form | | | Omega | Omega | Omega | Zigzag | Omega | Omega | Omega | Omega |
| Bulky | Initial | 512 | 853 | | 980 | 390 | 310 | 433 | 724 | 930 |
| | Comp ression | 9125 | 12530 | | 13100 | 11900 | 7850 | 8640 | 10200 | 11500 |
| | Resto ration | 4531 | 5310 | | 5932 | 4900 | 3320 | 4074 | 4810 | 5530 |

It can be verified that average initial bulkiness of the modified cross-section hollow composite fiber prepared in Examples 18 to 20 is about 781.6 and average initial bulkiness of the circular cross-section hollow composite fiber prepared in Comparative Examples 22 to 25 is about 366.8.

Further, as illustrated in FIG. 2, in the fiber according to the present invention, an omega (Ω)-shaped crimp was formed. It can be verified that average initial bulkiness in Examples 18 to 20 having the omega (Ω)-shaped crimp is about 781.6 and average initial bulkiness in Comparative Example 1 having a zigzag-shaped crimp is 310. That is, in order to have high bulkiness, it may be determined that it is preferred to have the omega (Ω)-shaped crimp.

Meanwhile, when the thermal insulation of the fiber according to the embodiment of the present invention was evaluated, like Table 11 below, it was tested that thermal insulation was excellent by a dead air layer ensured due to an interference effect of the volume control parts of the modified hollow fiber.

[Table 11]

| Classification | | Comparative Example 26 | Comparative Example 16 | Example 11 |
|---|---|---|---|---|
| Cross-sectional shape | | Circular cross-section | Circular hollow cross-section | Modified hollow cross-section |
| Hollow ratio (%) | | - | 21 | 22 |
| Thermal insulation ratio (%) | 100gsm | 71.3 | 75.2 | 81.5 |
| | 170gsm | 75.3 | 82.2 | 88.1 |
| | 250gsm | 82.3 | 86.7 | 93.2 |
| * gsm: Mean grams per square meter | | | | |

100 gsm is 100 g per 1 square meter, and as a gsm value is increased, grams are increased, a weight is increased, and a thickness is increased. The thickness of the nonwoven fabric per unit area is increased and a total sum of the dead air layers between the fibers is further increased to maximize thermal insulation.

The aforementioned present invention is not limited to the aforementioned exemplary embodiments and the accompanying drawings, and it will be obvious to those skilled in the technical field to which the present invention pertains that various substitutions, modifications, and changes may be made within the scope without departing from the technical spirit of the present invention.

**Claims**

1. A modified cross-section hollow fiber having a fiber center (M), comprising:

a hollow part (100), a shape maintaining part (200), and volume control parts (300),
wherein the volume control parts (300) have shape protruding away from the fiber center (M) and have respective end part with a rounded shape, wherein when the top of the end part of the volume control part (300) is defined as a peak (310) and a space between adjacent volume control parts (300) is defined as a valley (330), wherein a curvature radius of the peak is defined as R and a curvature radius of the valley is defined as r, and R and r values may be determined to be the same as or different from each other for each volume control part, and wherein a deviation of the curvature radius R of the peak and the curvature radius r of the valley is defined as Z, the following conditions are satisfied.

$$(1)\ -3 \le Z \le 4$$

$$(2)\ 0.9 \le \frac{\sum_{Z=1}^{N} Z}{} \le 1.8$$

Herein,

Z: R - r
N: The number of volume control parts (300).

2. The modified cross-section hollow fiber of claim 1, wherein the following condition is satisfied.

$$(3)\ (CT_{max} - R)/(CT_{min} - R) \geq 0.80$$

$$(4)\ (Ct_{max} - r)/(Ct_{min} - r) \geq 0.30$$

Herein,

T1: The largest value of a distance from a central point M to a peak (310)
T2: the smallest value of a distance from the central point M to the peak (310)
t1: the largest value of a distance from a central point M to a valley (330)
t2: the smallest value of a distance from the central point M to the valley (330)
CTmax: a circle formed by tangenting the outer peak of the volume control parts (300) having the second largest value of the distance between the center point M and the peak (310) based on T1
CTmin: a circle formed by tangenting the outer peak of the volume control part (300) having the second smallest value of the distance between the center point (M) and the peak (310) based on T2
Ctmax: a circle formed by tangenting the valley (310) from inside of the volume control part (300) having the second largest value of the distance between the center point (M) on t1
Ctmin: a circle formed by tangenting the valley (310) from inside of the volume control part (300) having the second smallest value of the distance between the center point (M) based on t2
CTmax-R: a difference value between the center point CTmaxM of CTmax and the center point (M)
CTmin-R: a difference value between the center point CTminM of CTmin and the center point (M)
Ctmax-r: a difference value between the center point CtmaxM of Ctmax and the center point (M)
Ctmin-r: a difference value between the center point CtminM of Ctmin and the center point (M)

3. The modified cross-section hollow fiber of claim 1, wherein a shape for forming the volume control part (300) is prepared by a radially deployed spinneret.

4. The modified cross-section hollow fiber of claim 3, wherein a radially deployed angle θ is 10 to 17° based on the center point (M).

5. The modified cross-section hollow fiber of claim 1, wherein the number of volume control parts (300) is 4 to 12.

6. The modified cross-section hollow fiber of claim 1, wherein a hollow ratio of the hollow part (100) is 15 to 30%.

7. Fibrous assemblies including the fiber according to any one of claims 1 to 6.

8. The fibrous assemblies of claim 7, wherein when the fibrous assemblies are prepared by a thermal bonding process, the fibrous assemblies include 60 to 90 wt% of a modified cross-section hollow fiber and 40 to 10 wt% of a bonding material, a length of the modified cross-section hollow fiber is 51 to 64 mm, and a thickness of the fiber is 0.44 to 0.89 tex (4 to 8 deniers).

9. The fibrous assemblies of claim 7, wherein when the fibrous assemblies are prepared by a melt blowing process, the fibrous assemblies include 20 to 60 wt% of a modified cross-section hollow fiber and 80 to 40 wt% of a fine PP fiber, a length of the modified cross-section hollow fiber is 32 to 51 mm, and a thickness of the fiber is 0.44 to 0.89 tex (4 to 8 deniers).

10. The fibrous assemblies of claim 7, wherein the modified cross-section hollow fiber includes a modified cross-section fiber structured to provide bulkiness and improve sound absorption of the assemblies simultaneously reducing diffraction effect of sound energy to be separated between adjacent fibers in the assemblies.

11. A sanitary nonwoven fabric including the modified cross-section hollow fiber according to any one of claims 1 to 6.

**12.** The sanitary nonwoven fabric of claim 11, wherein a modified cross-section hollow fiber in the nonwoven fabric is contained with 40 wt% or more.

**13.** The modified cross-section hollow fiber of claim 1, wherein the fiber is made of two types of polymers having different intrinsic viscosities as a composite fiber, and the complex-spun fiber has an omega ($\Omega$)-type spontaneous crimp.

**14.** The modified cross-section hollow fiber of claim 13, wherein the fiber has a side-by-side hollow structure.

**15.** The modified cross-section hollow fiber of claim 13, wherein the fiber is made of polyethylene terephthalate (PET), poly 1, 4-cyclohexylenedimethylene terephthalate (PCT), polypropylene (PP), nylon, and the like of two components having different viscosities.

**16.** The modified cross-section hollow fiber of claim 15, wherein the fiber is constituted by polyethylene terephthalate (PET) of two components having different viscosities and has 10 to 10,000 ppm of at least one polyfunctional group selected from a group consisting of polycarboxylic acid, polyol and polyoxycarboxylic acid in the fiber.

**17.** The modified cross-section hollow fiber of claim 13, wherein the crimp satisfies the following condition (5).

$$(5)\ 2.5\text{mm} \le \text{R'} \le 4.5\text{mm}$$

Herein, R': Curvature radius of circular arc of crimp

**18.** A nonwoven fabric for thermal insulation including the modified cross-section hollow fiber according to any one of claims 1 to 6.


**Patentansprüche**

**1.** Hohlfaser mit modifiziertem Querschnitt mit einem Faserzentrum (M), umfassend:

ein hohles Teil (100), ein formbeständiges Teil (200) und Volumenregelungsteile (300),
wobei die Volumenregelungsteile (300) eine Form haben, die von der Fasermitte (M) vorsteht und jeweils einen Endteil mit einer abgerundeten Form haben, - wobei, wenn die Oberseite des Endteils des Volumenregelungsteils (300) als eine Spitze (310) definiert ist und ein Raum zwischen benachbarten Volumenregelungsteilen (300) als ein Tal (330) definiert ist, wobei ein Krümmungsradius der Spitze als R definiert ist und ein Krümmungsradius des Tals als r definiert ist und R- und r-Werte so bestimmt werden können, dass sie für jedes Volumenkontrollteil gleich oder voneinander verschieden sind, und wobei eine Abweichung des Krümmungsradius R der Spitze und des Krümmungsradius r des Tals als Z definiert ist, die folgenden Bedingungen erfüllt sind.

$$(1) \qquad -3 \le Z \le 4$$

$$(2) \qquad 0{,}9 \le \sum_{Z=1}^{N} Z \le 1{,}8$$

wobei,

Z: R-r
N: Anzahl der Volumenregelungsteile (300).

**2.** Hohlfaser mit modifiziertem Querschnitt nach Anspruch 1, wobei die folgende Bedingung erfüllt ist.

$$(3) \qquad (CT_{max} - R)/(CT_{min} - R) \geq 0{,}80$$

$$(4) \qquad (Ct_{max} - r)/(Ct_{min} - r) \geq 0{,}30$$

wobei,

T1: Der größte Wert einer Distanz von einem zentralen Punkt M zu einer Spitze (310)
T2: der kleinste Wert einer Distanz vom Mittelpunkt M zur Spitze (310)
t1: der größte Wert einer Entfernung von einem zentralen Punkt M zu einem Tal (330)
t2: der kleinste Wert einer Entfernung vom Mittelpunkt M zum Tal (330) ist
CTmax: ein Kreis, der durch Tangentialisieren der äußeren Spitze der Volumenregelungsteile (300) mit dem zweitgrößten Wert des Abstands zwischen dem Mittelpunkt M und der Spitze (310) basierend auf T1 gebildet wird
CTmin: ein Kreis, der durch Tangentialisieren der äußeren Spitze des Volumenregelungteils (300) mit dem zweitkleinsten Wert des Abstands zwischen dem Mittelpunkt (M) und der Spitze (310) basierend auf T2 gebildet wird
Ctmax: ein Kreis, der durch Tangentialisieren des Tals (310) vom Inneren des Volumenregelungsteils (300) mit dem zweitgrößten Wert des Abstands zwischen dem Mittelpunkt (M) auf t1 gebildet wird
Ctmin: ein Kreis, der durch Tangentialisieren des Tals (310) vom Inneren des Volumenregelungsteils (300) mit dem zweitkleinsten Wert des Abstands zwischen dem Mittelpunkt (M) basierend auf t2 gebildet wird
CTmax-R: ein Differenzwert zwischen dem Mittelpunkt CTmaxM von CTmax und dem Mittelpunkt (M)
CTmin-R: ein Differenzwert zwischen dem Mittelpunkt CTminM von CTmin und dem Mittelpunkt (M)
Ctmax-r: ein Differenzwert zwischen dem Mittelpunkt CtmaxM von Ctmax und dem Mittelpunkt (M)
Ctmin-r: ein Differenzwert zwischen dem Mittelpunkt CtminM von Ctmin und dem Mittelpunkt (M)

3. Hohlfaser mit modifiziertem Querschnitt nach Anspruch 1, wobei eine Form zum Bilden des Volumenregelungsteils (300) durch eine radial entfaltete Spinndüse hergestellt wird.

4. Hohlfaser mit modifiziertem Querschnitt nach Anspruch 3, wobei der radial entfaltete Winkel θ 10 bis 17° beträgt, basierend auf den Mittelpunkt (M).

5. Hohlfaser mit modifiziertem Querschnitt nach Anspruch 1, wobei die Anzahl der Volumenregelungsteile (300) 4 bis 12 beträgt.

6. Hohlfaser mit modifiziertem Querschnitt nach Anspruch 1, wobei ein Hohlraumverhältnis des hohlen Teils (100) 15 bis 30% beträgt.

7. Faserzusammensetzungen einschließlich der Faser nach einem der Ansprüche 1 bis 6.

8. Faserzusammensetzungen nach Anspruch 7, wobei die Faserzusammensetzungen 60 bis 90 Gew.-% einer Hohlfaser mit modifiziertem Querschnitt und 40 bis 10 Gew.-% eines Bindematerials enthalten, eine Länge der Hohlfaser mit modifiziertem Querschnitt 51 bis 64 mm beträgt und die Dicke der Faser 0,44 bis 0,89 tex (4 bis 8 Denier) beträgt, wenn die Faserzusammensetzungen durch ein thermisches Bindeverfahren hergestellt werden.

9. Faserzusammensetzungen nach Anspruch 7, wobei die Faserzusammensetzungen 20 bis 60 Gew.-% einer Hohlfaser mit modifiziertem Querschnitt und 80 bis 40 Gew.-% einer feinen PP-Faser enthalten, eine Länge der Hohlfaser mit modifiziertem Querschnitt 32 bis 51 mm beträgt und die Dicke der Faser 0,44 bis 0,89 tex (4 bis 8 Denier) beträgt, wenn die Faserzusammensetzungen durch ein Schmelzblasverfahren hergestellt werden.

10. Faserzusammensetzungen nach Anspruch 7, wobei die Hohlfaser mit modifiziertem Querschnitt eine Faser mit modifiziertem Querschnitt umfasst, die so strukturiert ist, dass sie für Fülle sorgt und die Schallabsorption der Zusammensetzungen verbessert und gleichzeitig den Beugungseffekt der Schallenergie verringert, die zwischen benachbarten Fasern in den Zusammensetzungen zu trennen ist.

11. Hygienevliesstoff einschließlich der Hohlfaser mit modifiziertem Querschnitt nach einem der Ansprüche 1 bis 6.

12. Hygienevliesstoff nach Anspruch 11, wobei eine Hohlfaser mit modifiziertem Querschnitt in dem Vliesstoff mit 40 Gew.-% oder mehr enthalten ist.

13. Hohlfaser mit modifiziertem Querschnitt nach Anspruch 1, wobei die Faser aus zwei Arten von Polymeren mit unterschiedlichen intrinsischen Viskositäten als Verbundfaser hergestellt ist und die komplex gesponnene Faser eine spontane Kräuselung vom Omega ($\Omega$)-Typ aufweist.

14. Hohlfaser mit modifiziertem Querschnitt nach Anspruch 13, wobei die Faser eine nebeneinander liegende Hohlstruktur aufweist.

15. Hohlfaser mit modifiziertem Querschnitt nach Anspruch 13, wobei die Faser aus Polyethylenterephthalat (PET), Poly-1,4-cyclohexylendimethylenterephthalat (PCT), Polypropylen (PP), Nylon und dergleichen aus zwei Komponenten mit unterschiedlicher Viskosität hergestellt ist.

16. Hohlfaser mit modifiziertem Querschnitt nach Anspruch 15, wobei die Faser aus Polyethylenterephthalat (PET) aus zwei Komponenten mit unterschiedlichen Viskositäten besteht und 10 bis 10.000 ppm mindestens einer polyfunktionellen Gruppe, ausgewählt aus der Gruppe bestehend aus Polycarbonsäure, Polyol und Polyoxycarbonsäure in der Faser aufweist.

17. Hohlfaser mit modifiziertem Querschnitt nach Anspruch 13, wobei die Kräuselung die folgende Bedingung (5) erfüllt.

$$(5) \qquad 2,5 \text{ mm} \leq R' \leq 4,5 \text{ mm}$$

wobei, R': Krümmungsradius des Kräuselkreisbogens ist

18. Vliesstoff zur Wärmedämmung einschließlich der Hohlfaser mit modifiziertem Querschnitt nach einem der Ansprüche 1 bis 6.

**Revendications**

1. Fibre creuse à section transversale modifiée ayant un centre de la fibre (M), comprenant :

une partie creuse (100), une partie de maintien de forme (200), et des parties de contrôle du volume (300), dans laquelle les parties de contrôle du volume (300) ont une forme faisant saillie du centre de la fibre (M) et ont une partie d'extrémité respective ayant une forme ronde, - dans laquelle, lorsque le sommet de la partie d'extrémité de la partie de contrôle du volume (300) est défini comme une crête (310) et un espace entre les parties de contrôle du volume adjacentes (300) est défini comme une vallée (330), dans laquelle un rayon de courbure de la crête est défini comme R et un rayon de courbure de la vallée est défini comme r, et les valeurs R et r peuvent être déterminés pour être égales ou différentes l'une de l'autre pour chaque partie de contrôle du volume, et dans laquelle une déviation du rayon de courbure R de la crête et du rayon de courbure r de la vallée est définie comme Z, les conditions suivantes sont remplies.

$$(1) \ -3 \leq Z \leq 4$$

$$(2) \ 0,9 \leq \sum_{Z=1}^{N} Z \leq 1,8$$

Où,

Z : R - r
N : Le nombre de parties de contrôle du volume (300).

**2.** Fibre creuse à section transversale modifiée selon la revendication 1, dans laquelle la condition suivante est remplie.

$$(3)\ (CT_{max} - R)/(CT_{min} - R) \geq 0,80$$

$$(4)\ (Ct_{max} - r)/(Ct_{min} - r) \geq 0,30$$

Où,

T1 : La plus grande valeur d'une distance d'un point central M à une crête (310)
T2 : la plus petite valeur d'une distance du point central M à la crête (310)
t1 : la plus grande valeur d'une distance d'un point central M à une vallée (330)
t2 : la plus petite valeur d'une distance du point central M à la vallée (330)
CTmax : un cercle formé en déterminant une tangente à la crête externe des parties de contrôle du volume (300) ayant la deuxième plus grande valeur de la distance entre le point central M et la crête (310) basée sur T1
CTmin : un cercle formé en déterminant une tangente à la crête externe de la partie de contrôle du volume (300) ayant la deuxième plus petite valeur de la distance entre le point central (M) et la crête (310) basée sur T2
Ctmax : un cercle formé en déterminant une tangente à la vallée (310) de l'intérieur de la partie de contrôle du volume (300) ayant la deuxième plus grande valeur de la distance entre le point central (M) basée sur t1
Ctmin : un cercle formé en déterminant une tangente à la vallée (310) de l'intérieur de la partie de contrôle du volume (300) ayant la deuxième plus petite valeur de la distance entre le point central (M) basée sur t2
CTmax-R : une valeur de différence entre le point central CTmaxM de CTmax et le point central (M)
CTmin-R : une valeur de différence entre le point central CTminM de CTmin et le point central (M)
Ctmax-r : une valeur de différence entre le point central CtmaxM de Ctmax et le point central (M)
Ctmin-r : une valeur de différence entre le point central CtminM de Ctmin et le point central (M)

**3.** Fibre creuse à section transversale modifiée selon la revendication 1, dans laquelle une forme pour la formation de la partie de contrôle du volume (300) est préparée par une filière radialement déployée.

**4.** Fibre creuse à section transversale modifiée selon la revendication 3, dans laquelle un angle radialement déployé θ est compris entre 10 et 17θ en partant du point central (M).

**5.** Fibre creuse à section transversale modifiée selon la revendication 1, dans laquelle le nombre de parties de contrôle du volume (300) est compris entre 4 et 12.

**6.** Fibre creuse à section transversale modifiée selon la revendication 1, dans laquelle un rapport creux de la partie creuse (100) est compris entre 15 et 30%.

**7.** Ensembles de fibres incluant la fibre selon l'une quelconque des revendications 1 à 6.

**8.** Ensembles de fibres selon la revendication 7, dans lesquels, lorsque les ensembles de fibres sont préparés par un processus de collage thermique, les ensembles de fibres incluent de 60 à 90% en poids d'une fibre creuse à section transversale modifiée et de 40 à 10% en poids d'un matériau de liaison, une longueur de la fibre creuse à section transversale modifiée est comprise entre 51 et 64 mm, et une épaisseur de la fibre est comprise entre 0,44 et 0,89 tex (de 4 à 8 deniers).

**9.** Ensembles de fibres selon la revendication 7, dans lesquels, lorsque les ensembles de fibres sont préparés par un procédé de soufflage à l'état fondu, les ensembles de fibres incluent de 20 à 60% en poids d'une fibre creuse à section transversale modifiée et de 80 à 40% en poids d'une fibre PP fine, une longueur de la fibre creuse à section transversale modifiée est comprise entre 32 et 51 mm, et une épaisseur de la fibre est comprise entre 0,44 et 0,89 tex (de 4 à 8 deniers).

**10.** Ensembles de fibres selon la revendication 7, dans lesquels la fibre creuse à section transversale modifiée inclut une fibre à section transversale modifiée structurée pour fournir voluminosité et améliorer l'absorption acoustique des ensembles en réduisant simultanément l'effet de la diffraction de l'énergie sonore pour qu'elle soit séparée entre les fibres adjacentes dans les ensembles.

**11.** Textile non-tissé sanitaire incluant la fibre creuse à section transversale modifiée selon l'une quelconque des revendications 1 à 6.

**12.** Textile non-tissé sanitaire selon la revendication 11, dans lequel une fibre creuse à section transversale modifiée dans le textile non-tissé est présente à 40% en poids ou plus.

**13.** Fibre creuse à section transversale modifiée selon la revendication 1, dans laquelle la fibre est composée de deux types de polymères ayant différentes viscosités intrinsèques comme fibre composite, et la fibre filée en complexe a une frisure spontanée de type oméga (Ω).

**14.** Fibre creuse à section transversale modifiée selon la revendication 13, dans laquelle la fibre a une structure creuse en parallèle.

**15.** Fibre creuse à section transversale modifiée selon la revendication 13, dans laquelle la fibre est composée de polytéréphtalate d'éthylène (PET), 1,4-téréphtalate de polycyclohexylène-diméthylène (PCT), polypropylène (PP), nylon, et similaires de deux composants ayant des viscosités différentes.

**16.** Fibre creuse à section transversale modifiée selon la revendication 15, dans laquelle la fibre est constitué de polytéréphtalate d'éthylène (PET) de deux composants ayant des viscosités différentes et elle a de 10 à 10 000 ppm d'au moins un groupe polyfonctionnel sélectionné parmi un groupe composé d'acide polycarboxylique, polyol et acide polyoxycarboxylique dans la fibre.

**17.** Fibre creuse à section transversale modifiée selon la revendication 13, dans laquelle la frisure remplie la condition suivante (5).

$$(5) \; 2,5mm \le R' \le 4,5mm$$

Où, R' : Rayon de courbure de l'arc circulaire de frisure

**18.** Textile non-tissé d'isolation thermique incluant la fibre creuse à section transversale modifiée selon l'une quelconque des revendications 1 à 6.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

**REFERENCES CITED IN THE DESCRIPTION**

**Patent documents cited in the description**

- KR 1387465
- KR 20110069474
- JP H08246225 B
- US 2009246492 A